# EUROPEAN PATENT APPLICATION

(11) **EP 4 130 261 A1**
(43) Date of publication of application: **08.02.2023**
(21) Application number: 21919838.9
(22) Date of filing: 20.10.2021
(51) Int. Cl.: C12N 15/10, C12Q 1/6806, C12Q 1/686

(54) **BUFFER COMPOSITION FOR NUCLEIC ACID ISOLATION FOR COLUMN-BASED ONE-TUBE PCR, AND USE THEREOF**

(30) Priority: 15.01.2021 KR 20210005968
(71) Applicant: Gene2us Corp., Chungcheongnam-do 31214 (KR)
(72) Inventor: RYU, Seong Ho, Cheonan-si Chungcheongnam-do 31163 (KR)
(74) Representative: LLR
(86) International application number: PCT/KR2021/014744
(87) International publication number: WO 2022/154214

(57) **Abstract**

The present invention relates to a nucleic acid isolation buffer composition, a nucleic acid isolation kit in which a membrane filter comprising same is provided, and a nucleic acid isolation method, wherein the nucleic acid isolation buffer composition has excellent compatibility with membrane filters of various materials, and enables the excellent isolation of nucleic acids from various samples, and, particularly, a nucleic acid isolation kit comprising same enables column-based one-tube PCR, and a nucleic acid isolation method using same enables the direct application of the membrane filter, on which nucleic acids are immobilized, to PCR without a separate elution process. Therefore, the nucleic acid isolation composition, the kit and the isolation method according to the present invention can be effectively used in rapid diagnosis onsite.

## Description

### [Technical Field]

The present invention relates to a nucleic acid isolation buffer composition for rapidly and easily isolating nucleic acids, a nucleic acid isolation kit including the same, and a nucleic acid isolation method, and retains technical features enabling column-based one-tube PCR.

### [Background Art]

In general, in order to diagnose various diseases and test genetically recombinant plants, a process of purifying nucleic acids from samples such as tissues, blood, hair roots, cells, plants, and strains suitable for the purpose of the test is first performed.

A method for quickly and rapidly isolating nucleic acids from biological substances is of utmost importance in a diagnostic process. Accordingly, various studies on effective and reproducible methods for selectively isolating only nucleic acids from various types of substances contained in cell lysis solutions have been made.

A commonly used nucleic acid purification method includes culturing the collected sample in a lysis buffer for a certain period of time to destroy cell walls and cell membranes, and then perform a pretreatment process of lysing nucleic acids to expose the nucleic acids, and removing the destroyed impurities.

In more detail, a sample is put into a lysis buffer, incubated, and pre-treated, and then put into a spin column with a membrane to which a nucleic acid is bound. The column to which the pretreated nucleic acid sample is put is alternately centrifuged and washed multiple times to bind the nucleic acid to an inner membrane of the spin column. Pure nucleic acids are isolated by performing a process of isolating the nucleic acid from a membrane using a TE buffer (T ten E one buffer), an elution buffer, and water along with a drying process using the membrane to which the nucleic acid is bound.

Accordingly, the conventional nucleic acid isolation method using a column goes through a pretreatment process, a centrifugation process, a washing process, a drying process, and a gene isolation process. Hence, various devices such as a pretreatment buffer, a centrifuge, a drying device and an isolation buffer, and a tool such as a micropipette is required. In addition, a lot of time and manpower are required as samples are moved to perform each process. In addition, a plurality of washing processes for binding nucleic acids to a membrane or washing impurities also contributes to an increase in the time required. In addition, in order to utilize the obtained nucleic acid for PCR reaction, a very small amount of a nucleic acid sample in a µl unit needs to be obtained using a specific tool (micropipette). Hence, it is difficult to perform nucleic acid extraction and subsequent PCR reactions in places outside a laboratory.

Since the nucleic acid isolation method using a column has a very low level of proficiency required by a user and thus has high usefulness, it is very important to prepare a plan that may be utilized by solving the aforementioned issues.

In the meantime, over the past 20 years, mankind has experienced viral infectious diseases such as SARS-CoV (Severe acute respiratory syndrome-Coronavirus (2002-2003)) and H1N1 new influenza (2009), which cause severe respiratory syndrome. Recently, in 2012 and 2015, the MERS coronavirus, which causes Middle East respiratory syndrome (MERS), was a regional epidemic in Saudi Arabia and South Korea.

Moreover, on December 31, 2019, the 2019 novel Coronavirus (2019-nCoV; COVID-19) first reported to the World Health Organization (WHO) occurred. This viral infectious disease spreads very fast, so the number of patients is continuously and quickly increasing, and very fatal damage is occurring.

Accordingly, although rapid diagnosis onsite is of utmost importance, the conventional nucleic acid isolation technology using a column has the aforementioned issues, and thus the utilization onsite is low. Accordingly, it is necessary to develop a rapid and easy nucleic acid isolation technology that shows an improvement.

### [Disclosure]

### [Technical Problem]

The present inventors have prepared a novel nucleic acid isolation buffer composition for isolating nucleic acids and a column-based nucleic acid isolation kit in which a membrane filter including the same is provided, and identified that it was possible to quickly and accurately isolate nucleic acids from various samples when PCR was performed by separately extracting a membrane filter on which nucleic acids were immobilized or one-tube PCR, which is directly applied to PCR using the same. Therefore, the present inventors completed the present invention by developing a nucleic acid isolation technology that does not require a separate centrifugation, washing process, and drying process.

Accordingly, a purpose of the present disclosure is to provide a one-step nucleic acid isolation buffer composition applicable to column-based one-tube PCR.

Another purpose of the present disclosure is to provide a one-step nucleic acid isolation kit.

Another purpose of the present disclosure is to provide a method for isolating nucleic acids from a sample.

Another purpose of the present disclosure is to provide a method for amplifying nucleic acids.

### [Technical Solution]

In order to achieve the above purpose, the present disclosure provides a one-step nucleic acid isolation buffer composition including ethanol, SDS (sodium dodecyl sulfate), sodium chloride (NaCl), EDTA (ethylenediaminetetraacetic acid) and Tris-HCl.

Further, the present disclosure provides a one-step nucleic acid isolation kit including a nucleic acid isolation buffer composition according to the present invention and a column in which a membrane filter is provided.

Further, the present disclosure provides a method for isolating nucleic acids from a sample using the nucleic acid isolation kit according to the present invention.

Further, the present disclosure provides a method for amplifying nucleic acids, in which the method includes performing PCR using the nucleic acid isolated by the method according to the present invention as a template.

### [Advantageous Effects]

A nucleic acid isolation buffer composition, a nucleic acid isolation kit in which a membrane filter including the same is provided, and a nucleic acid isolation method according to the present invention enable quick and accurate isolation of nucleic acids from a variety of samples in one-step. In particular, they can be directly applied to PCR by putting a PCR reaction mix in a column including a membrane filter on which nucleic acids are immobilized without a separate elution process. The conventional nucleic acid isolation method using a column is performed through a complicated process, and accordingly, various devices and tools are required, and excessive time, capital, and manpower are required. Moreover, there is a risk of some loss or contamination of the isolated nucleic acids as each phase is performed. In contrast, since the nucleic acid isolation composition, kit and isolation method according to the present invention can be applied to a column-based one-tube PCR method, a special device is not required, and nucleic acids can be easily and quickly isolated. Moreover, there is little risk of loss or contamination of the isolated nucleic acids, and it can be provided at a low price. In addition, PCR or RT-PCR can also be performed by isolating the membrane filter on which the nucleic acids are immobilized from the column and putting the same in a separate tube. Accordingly, it can be applied to various PCR methods, and thus can be usefully utilized for rapid diagnosis onsite because of its high utilization.

### [Description of Drawings]

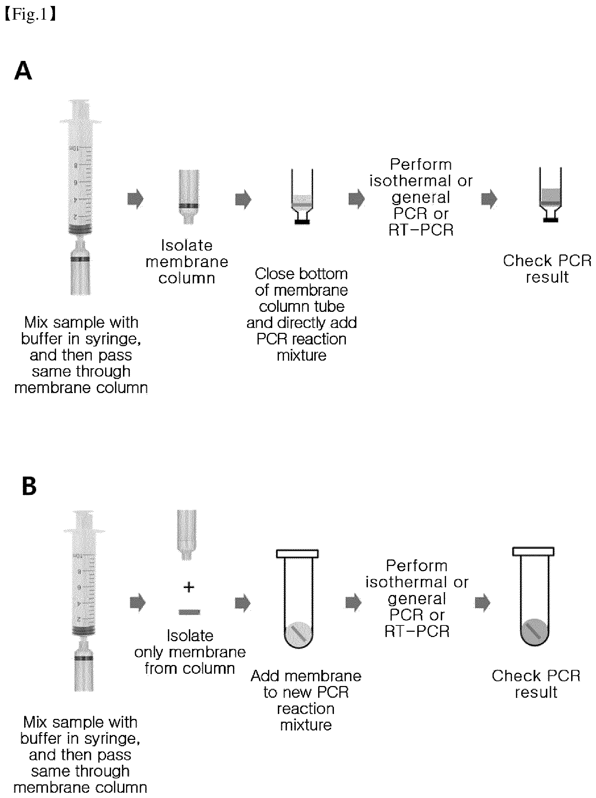

FIG. 1A is an image showing a process of performing PCR (including isothermal PCR, general PCR or RT-PCR) by directly applying a membrane filter on which the isolated nucleic acids are immobilized using a syringe provided with a membrane filter and a nucleic acid isolation buffer composition according to an embodiment of the present invention. FIG. 1B is an image showing a process of performing PCR (including isothermal PCR, general PCR or RT-PCR) using the same after isolating the membrane on which the nucleic acids are immobilized from a column, and extracting the nucleic acids with an elution solution.
FIG. 2 is a view identifying the optimal concentrations of SDS and EtOH in a nucleic acid isolation method using the nucleic acid isolation buffer composition according to an embodiment of the present invention and directly applying the membrane filter on which the nucleic acids are immobilized to PCR, excluding an elution process.
FIG. 3 is a view identifying the pore size of a compatible membrane filter in the compatibility of the nucleic acid isolation buffer composition of an embodiment of the present invention and a commercial membrane filter.
FIG. 4 is a view identifying the compatibility of the nucleic acid isolation buffer composition of an embodiment of the present invention and a silica membrane filter.
FIG. 5 is a view identifying the compatibility of the nucleic acid isolation buffer composition of an embodiment of the present invention and a glass microfiber filter.
FIG. 6 is a view identifying whether gDNA is immobilized on the glass fiber filter after the nucleic acids are isolated using a syringe provided with a glass fiber filter and a nucleic acid isolation buffer composition of an embodiment of the present invention.
FIG. 7 is a view identifying the optimal size compatible for the PCR reaction of the glass fiber filter in the use of the glass fiber filter and the nucleic acid isolation buffer composition of an embodiment of the present invention.
FIG. 8 is a view identifying the sensitivity according to an amount of gDNA in PCR to which a glass fiber filter on which nucleic acids isolated in one step are immobilized using the nucleic acid isolation buffer composition according to an embodiment of the present invention is applied.
FIG. 9 is a view identifying the sensitivity according to the number of cells from which nucleic acids are to be isolated in PCR to which a glass fiber filter on which nucleic acids isolated in one step are immobilized using the nucleic acid isolation buffer composition according to an embodiment of the present invention is applied.
FIG. 10 is a view identifying the efficiency of bacterial gDNA (bacteria genomic DNA) isolation in PCR to which a glass fiber filter on which nucleic acids isolated in one step are immobilized using the nucleic acid isolation buffer composition according to an embodiment of the present invention is applied.
FIG. 11 is a view identifying the RNA isolation efficiency according to pH in PCR to which a glass fiber filter on which nucleic acids isolated in one step are immobilized using the nucleic acid isolation buffer composition according to an embodiment of the present invention is applied.
FIG. 12 is a view identifying the PCR reaction according to pH in PCR using the nucleic acid isolation buffer composition according to an embodiment of the present invention and directly applying the glass fiber filter on which the nucleic acids are immobilized, excluding the elution process.

### [Modes of the Invention]

Hereinafter, the present invention will be described in detail.

The present invention provides a one-step nucleic acid isolation buffer composition including ethanol, sodium dodecyl sulfate (SDS), sodium chloride (NaCl), ethylenediaminetetraacetic acid (EDTA), and Tris-HCl.

The technical feature of the nucleic acid isolation buffer composition according to the present invention resides in isolating nucleic acids in one-step, and applying the same to column-based one-tube PCR to proceed from nucleic acid isolation to PCR reaction in a one tube. The present invention is directed to addressing an issued associated with a complicated isolation method that requires various solutions of the conventional nucleic acid isolation method using a column, the need for additional equipment, and excessive waste of time, resources, and manpower.

In particular, in the gDNA isolation method, a large amount of salt (NaCl, etc.) and EtOH are required for cell lysis and binding of a membrane filter to the gDNA, which may cause an issue that the PCR result is affected by the salt and EtOH. Accordingly, when PCR is directly performed using the membrane filter on which the isolated nucleic acids are immobilized after one-step isolation of nucleic acids using the nucleic acid isolation buffer composition of the present invention. Since there is no precise isolation process and separate washing process using an elution solution, it is very important to have a composition that may minimize the effect of the salt and EtOH lysed in the membrane filter on the PCR result.

In an embodiment of the present invention, in one-step DNA isolation using the composition of the present invention, the optimal conditions of SDS and EtOH were identified. As a result, it was found that the composition including 50% (v/v) or less of EtOH and 1% (w/v) or less of SDS could isolate nucleic acids excellently.

Accordingly, in the one-step nucleic acid isolation buffer composition of the present invention, the ethanol may be included in an amount of 10% (v/v) to 50% (v/v), 10% (v/v) to 40% (v/v), 10% (v/v) to 30% (v/v), 10% (v/v) to 20% (v/v), 20% (v/v) to 50% (v/v), 20% (v/v) to 40% (v/v), 20% (v/v) to 30% (v/v), 30% (v/v) to 50% (v/v), 30% (v/v) to 40% (v/v), or 40% (v/v) to 50% (v/v), and more preferably 20% (v/v) to 50% (v/v). When the ethanol is included at a concentration exceeding 50% (v/v), a partial gDNA isolation may be achieved, but when a buffer is stored at room temperature, an issue of NaCl crystallization occurs.

In addition, the SDS may be included in an amount of 0.1% (w/v) to 0.9% (w/v), 0.1% (w/v) to 0.8% (w/v), 0.1% (w/v) to 0.7% (w/v), 0.1% (w/v) to 0.6% (w/v), 0.1% (w/v) to 0.5% (w/v), 0.1% (w/v) to 0.4% (w/v), 0.1% (w/v) to 0.3% (w/v), 0.1% (w/v) to 0.2% (w/v), 0.2% (w/v) to 0.9% (w/v), 0.2% (w/v) to 0.8% (w/v), 0.2% (w/v) to 0.7% (w/v), 0.2% (w/v) to 0.6% (w/v), 0.2% (w/v) to 0.5% (w/v), 0.2% (w/v) to 0.4% (w/v), 0.2% (w/v) to 0.3% (w/v), 0.3% (w/v) to 0.9% (w/v), 0.3% (w/v) to 0.8% (w/v), 0.3% (w/v) to 0.7% (w/v), 0.3% (w/v) to 0.6% (w/v), 0.3% (w/v) to 0.5% (w/v), 0.3% (w/v) to 0.4% (w/v), 0.4% (w/v) to 0.9% (w/v), 0.4% (w/v) to 0.8% (w/v), 0.4% (w/v) to 0.7% (w/v), 0.4% (w/v) to 0.6% (w/v), 0.4% (w/v) to 0.5% (w/v), 0.5% (w/v) to 0.9% (w/v), 0.5% (w/v) to 0.8% (w/v), 0.5% (w/v) to 0.7% (w/v), 0.5% (w/v) to 0.6% (w/v), 0.6% (w/v) to 0.9% (w/v), 0.6% (w/v) to 0.8% (w/v), 0.6% (w/v) to 0.7% (w/v), 0.7% (w/v) to 0.9% (w/v), 0.7% (w/v) to 0.8% (w/v), or 0.8% (w/v) to 0.9% (w/v), and more preferably 0.1% (w/v) to 0.5% (w/v).

The sodium chloride (NaCl) may be included in an amount of 0.1 M to 1 M, 0.1 M to 0.9 M, 0.1 M to 0.8 M, 0.1 M to 0.7 M, 0.1 M to 0.6 M, 0.1 M to 0.5 M, 0.1 M to 0.4 M, 0.1 M to 0.3 M, 0.1 M to 0.2 M, 0.2 M to 1 M, 0.2 M to 0.9 M, 0.2 M to 0.8 M, 0.2 M to 0.7 M, 0.2 M to 0.6 M, 0.2 M to 0.5 M, 0.2 M to 0.4 M, 0.2 M to 0.3 M, 0.3 M to 1 M, 0.3 M to 0.9 M, 0.3 M to 0.8 M, 0.3 M to 0.7 M, 0.3 M to 0.6 M, 0.3 M to 0.5 M, 0.3 M to 0.4 M, 0.4 M to 1 M, 0.4 M to 0.9 M, 0.4 M to 0.8 M, 0.4 M to 0.7 M, 0.4 M to 0.6 M, 0.4 M to 0.5 M, 0.5 M to 1 M, 0.5 M to 0.9 M, 0.5 M to 0.8 M, 0.5 M to 0.7 M, 0.5 M to 0.6 M, 0.6 M to 1 M, 0.6 M to 0.9 M, 0.6 M to 0.8 M, 0.6 M to 0.7 M, 0.7 M to 1 M, 0.7 M to 0.9 M, 0.7 M to 0.8 M, 0.8 M to 1 M, 0.8 M to 0.9 M, or 0.9 M to 1 M, and more preferably 0.5 M to 1 M.

The EDTA may be included in an amount of 1 mM to 100 mM, and the Tris-HCl may be included in an amount of 1 mM to 50 mM.

The one-step nucleic acid isolation buffer composition of an embodiment of the present invention may include, as a preferred embodiment, 20% (v/v) to 50% (v/v) of ethanol, 0.1% (w/v) to 0.5% (w/v) of SDS, 0.5 M to 1 M of sodium chloride (NaCl), 1 mM to 100 mM of EDTA, and 1 mM to 50 mM of Tris-HCl.

In addition, preferably, the buffer composition may include 1 to 5 parts by weight of 20% (v/v) to 50% (v/v) ethanol, 1 to 5 parts by weight of 0.1% (w/v) to 0.5% (w/v) SDS, 2.5 to 10 parts by weight of 0.5 M to 1 M sodium chloride (NaCl), 1 to 5 parts by weight of 1 mM to 100 mM EDTA, and 1 to 5 parts by weight of 1 mM to 50 mM Tris-HCl, with respect to 100 parts by weight of the buffer composition. More preferably, the buffer composition may include 2.5 parts by weight of 20% (v/v) to 50% (v/v) ethanol, 2.5 parts by weight of 0.1% (w/v) to 0.5% (w/v) SDS, 5 parts by weight of 0.5 M to 1 M sodium chloride (NaCl), 2.5 parts by weight of 1 mM to 100 mM EDTA, and 2.5 parts by weight of 1 mM to 5 mM Tris-HCl, with respect to 100 parts by weight of the buffer composition.

In an embodiment of the present invention, as a more preferred embodiment, an aqueous solution including 50% (v/v) of ethanol, 0.25% (w/v) of SDS, 0.25M of sodium chloride (NaCl), 12.5 mM of EDTA, and 25 mM of Tris-HCl was prepared.

When gDNA is isolated using the nucleic acid isolation buffer composition according to the present invention, it is preferable that the Tris-HCl is pH 8.

In addition, according to an embodiment of the present invention, it was identified that when total RNA is isolated using the nucleic acid isolation buffer composition according to an embodiment of the present invention, it is preferable to perform the isolation at pH 4 to 7.

Accordingly, in the case of isolating total RNA using the nucleic acid isolation buffer composition of the present invention, it is preferable that the pH of the Tris-HCl is pH 4 to 7.

In addition, an embodiment of the present invention provides a one-step nucleic acid isolation kit including the one-step nucleic acid isolation buffer composition according to the present invention, and a column in which a membrane filter is provided.

In addition, the one-step nucleic acid isolation kit of the present invention may be in a form that may remove a filtrate from a lower portion after applying pressure to an upper portion of the column to filter a solution through the membrane filter, and more preferably in a form of a syringe. When the kit is manufactured in the form of a syringe, there is no need for a separate tool such as a centrifuge, so it has the benefit of being portable and enabling an onsite direct detection.

The membrane filter may be any one selected from the group consisting of polyvinylidene fluoride (PVDF), nylon filter, cellulose nitrate filter, paper filter, glass fiber filter, and silica filter. In the following examples, it was identified that the compatibility between the one-step nucleic acid isolation buffer composition of the present invention and each membrane filter was excellent.

According to an embodiment of the present invention, in DNA isolation using the nucleic acid isolation buffer composition of the present invention and a commercial membrane filter made of a PVDF substance, it was identified that the filter having the pore size of 0.1 µm showed the highest DNA isolation efficiency.

Meanwhile, the negatively charged membrane filters, such as PVDF membrane, silica membrane and glass fiber membrane filters, require a large amount of salt (0.1 to 1 M NaCl) as an intermediate medium of positive charge to capture negatively charged gDNA. Accordingly, when PCR is performed after adding the PCR reaction mixture to the glass fiber filter in which the gDNA is captured without separate purification, the inhibition of the PCR reaction may be caused due to NaCl lysed from the glass fiber filter. In another embodiment of the present invention, the optimal size of the glass fiber filter was identified in the utilization with the nucleic acid isolation buffer composition of the present invention. As a result, it was identified that a glass fiber filter with a size of 2 × 2 mm² or less per 50 µl of total volume was optimal (based on 0.25 M NaCl).

The nucleic acid may be any one or more selected from the group including deoxyribonucleic acid (DNA), ribonucleic acid (RNA), and pentose nucleic acid (PNA).

In addition, an embodiment of the present invention provides a method for isolating nucleic acids from a sample using the nucleic acid isolation kit according to the present invention.

In addition, an embodiment of the present invention provides a method for amplifying nucleic acids, in which the method includes performing PCR using the nucleic acids isolated through the nucleic acid isolation method of the present invention as a template.

More specifically, the method may be performed by: dissolving a sample by immersing the same in a nucleic acid isolation buffer according to the present invention; incubating a solution in which the sample is dissolved at room temperature; transferring the solution in which the sample is dissolved to a column provided with a membrane filter; and applying pressure to the column to immobilize nucleic acids on a filter and removing a filtrate, or performed by dissolving a sample by immersing the same in a nucleic acid isolation buffer according to the present invention; incubating a solution in which the sample is dissolved at room temperature; transferring the solution in which the sample is dissolved to a column provided with a membrane filter; applying pressure to the column to immobilize nucleic acids to the filter and removing a filtrate; putting an elution solution in the column on which the nucleic acids are immobilized and passing the same through the membrane filter; and obtaining the elution solution.

In the method for amplifying the nucleic acids according to the present invention, a nucleic acid serving as a template may be used directly using a filter on which nucleic acids are immobilized, or may be used in a form included in an elution solution.

In this connection, when directly using the filter in which the nucleic acids are immobilized, PCR may be performed by directly adding a PCR reaction mixture to a column provided with a filter on which the nucleic acids are immobilized, as shown in FIGS. 1A and 1B, or PCR may be performed by isolating the filter on which the nucleic acids are immobilized, transferring the same to a separate tube, and then reacting the same with the PCR reaction mixture. In this connection, the filter on which the nucleic acids is immobilized may be cut to an appropriate size.

In this connection, the PCR reaction is a method for identifying the isolated nucleic acids, and any method known in the art is applicable as long as it is a method capable of identifying the isolated nucleic acids. Preferably, it may be a polymerase chain reaction (PCR), a reverse transcription polymerase chain reaction (RT-PCR), and an isothermal amplification PCR, but is not limited thereto.

The elution solution may be used without limitation as long as it is an elution buffer known in the art. Preferably, it may be any one selected from the group consisting of distilled water, TE buffer (T ten E one buffer), and a mixture thereof, but is not limited thereto. In addition, the elution solution known in the art may be a TE buffer (10 mM Tris-HCl, pH 8.0, 0.1 mM EDTA) or an elution buffer (10 mM Tris-HCl, pH 7.5 to 8.5), but is not limited thereto.

In the nucleic acid isolation method of the present invention, the incubation may be performed for 2 to 10 minutes, more preferably for 5 minutes, as a process of lysing cells.

The incubation may be performed after immersing a sample in 1 to 5 ml of the nucleic acid isolation buffer according to the present invention, shaking and mixing the same, but is not limited thereto.

In addition, the sample may be a biological or non-biological sample.

The biological sample is a sample including DNA and RNA, and may be any one or more selected from the group consisting of nasal aspirate, bronchial aspirate, organ secretions, sputum, tears, saliva, cell, cell extract, whole blood, plasma, serum, mucus, nasal washes, urine, semen, peritoneal washings, ascites, cystic fluid, meningeal fluid, amniotic fluid, leukocytes, peripheral blood mononuclear cells, buffy coat, glandular fluid, pancreatic fluid, lymph fluid, pleural fluid, nipple aspirate, synovial fluid, joint aspirate, and cerebrospinal fluid, but is not limited thereto.

In addition, the non-biological sample may include chemically synthesized PNA.

The technical feature of the nucleic acid isolation buffer composition, the nucleic acid isolation kit in which the membrane filter including the same is provided, and the nucleic acid isolation method according to the present invention resides in the excellent isolation of nucleic acids from various samples in one-step, and, particularly, in the direct application of the membrane filter, on which nucleic acids are immobilized, to PCR without a separate elution process.

The present invention is directed to an excellent improvement in issues such as excessive time, capital and manpower required, and the risk of loss or contamination of the isolated nucleic acids in the conventional nucleic acid isolation method using a column, and to quickly isolating the nucleic acids and utilizing the same for rapid diagnosis onsite.

The above-mentioned contents of the present invention are applied identically to each other unless they contradict each other, and appropriate modifications thereof by those of ordinary skill in the art may be included in the scope of the present invention.

Hereinafter, the present invention will be described in detail through the examples, but the scope of the present invention is not limited only to the following examples.

### Example 1. Preparation of Nucleic Acid Isolation Buffer Composition

As a preferred embodiment of the buffer composition for nucleic acid isolation, the present inventors prepared an aqueous solution including 2.5 ml of 50% (v/v) ethanol, 2.5 ml of 0.25% (w/v) SDS, 5 ml of 0.25 M sodium chloride (NaCl), 2.5 ml of 12.5 mM EDTA, and 2.5 ml of 25 mM Tris-HCl, with respect to 100 ml of the buffer composition

In the meantime, the conventional nucleic acid isolation method is performed through a complicated process of a pretreatment process, a centrifugation process, a washing process, a drying process, and a gene isolation process. To this end, a lysis buffer, a binding buffer, a washing buffer, and an elution buffer are required for each phase, and a special device such as a centrifuge is also required, which requires excessive time, capital and manpower. Moreover, there is a risk of some loss or contamination of the isolated nucleic acids as each phase is performed. In addition, in order to perform PCR using additionally isolated nucleic acids, it is necessary to collect a very small amount of about 1 to 5 µl of the isolated DNA using a micropipette and pipette tips. It is difficult to perform the same onsite where there is no such tool. On the other hand, the nucleic acid isolation buffer composition of an embodiment of the present invention prepared in Example 1 is a composition in which all components are mixed into one. The nucleic acid isolation is possible in one-step. Hence, it is possible to rapidly isolate the nucleic acids, and moreover, there is little risk of loss or contamination of the isolated nucleic acids. Since there is no additional equipment required therefor, real-time detection is possible more easily and quickly onsite.

### Example 2. Nucleic Acid Isolation Method using Nucleic Acid Isolation Buffer Composition

### 2-1. Nucleic Acid Isolation Method excluding Elution Process

In the nucleic acid isolation method of an embodiment of the present invention, the result may be immediately checked by directly applying the filter on which the nucleic acids are immobilized to PCR without a separate elution process.

First, a sample was collected using a cotton swab, immersed in 2 ml of the nucleic acid isolation buffer composition prepared in Example 1, shaken and mixed, and then incubated at room temperature for 5 minutes to lyse the cells.

After the incubation was completed, the solution was transferred to a syringe provided with a membrane filter, and the solution was passed through the membrane filter by slowly applying pressure to immobilize the nucleic acids and remove the filtrate. Then, PCR was performed using a membrane filter on which the nucleic acids were immobilized.

More specifically, the PCR reaction mixture (25 µl of 2X i-TaqTM PCR mix, 1 µl of 10 pmol/µl forward primer, 1 µl of 10 pmol/µl reverse primer, 23 µl of the distilled water, total volume 50 µl) was added to a syringe including a membrane filter on which the nucleic acids were immobilized. Then, PCR reaction was performed, which was performed under the following PCR conditions: initial denaturation: 94°C, 2 minutes, 30 cycles (denaturation: 94°C, 20 seconds; annealing: 55°C, 10 seconds; extension: 72°C, 30 seconds; final extension: 72°C, 5 minutes).

### 2-2. Nucleic Acid Isolation Method including Elution Process

In addition, after eluting the membrane filter on which the nucleic acids are immobilized using an elution solution, it may be applied to PCR.

The phases were performed in the same manner as in 2-1 above until the nucleic acids were immobilized on the membrane filter. Distilled water was put into a syringe from which a filtrate was removed and passed through a membrane filter to obtain the same. In this connection, it may be washed once with 100% EtOH to selectively filter out impurities before adding the distilled water. The determination of whether DNA or RNA was included in the finally obtained solution was identified in the same manner as in Example 2-1.

### Example 3. Identification of Optimal Conditions for One-Step Nucleic Acid Isolation Buffer Composition

In gDNA isolation, a large amount of salt is required for cell lysis and binding of gDNA to the membrane filter, and EtOH also needs to be contained. Accordingly, when the nucleic acids are isolated in one-step using the nucleic acid isolation buffer composition according to an embodiment of the present invention, and then PCR is directly performed using the membrane filter on which the nucleic acids are immobilized, a composition capable of minimizing the influence of the salt and EtOH is very important.

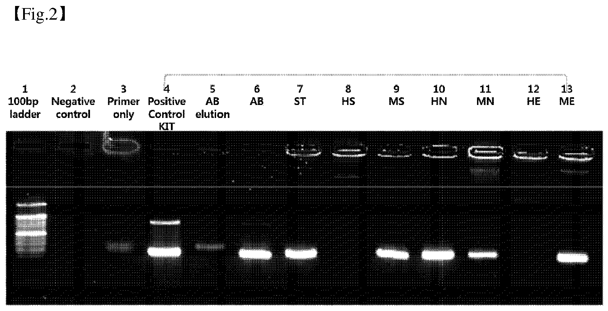

In order to identify the above, the DNA isolation efficiency of the nucleic acid isolation buffer composition including the components SDS (0.5% or 1%), NaCl (0.5 M or 1 M), or EtOH (50% or 70%), respectively, was identified. The results are shown in FIG. 2. In this connection, according to each experimental group, it was performed in the same manner as in Example 2-1 or 2-2. The glass microfiber filters (GF/C, Whatman co) were used as membrane filters, and 293T cells (a human cell line derived from the HEK 293 cell line expressing a mutation of the SV40 large T antigen) were used identically at 1.75 × 10⁹. 5 µl of the isolated sample and 1 µl of dye was mixed and loaded on 0.8% agarose gel, and the results were identified using GelDoc (electrophoretic imaging system).

In FIG. 2, line 1 is a 100 bp ladder, line 2 is a negative control group (PCR is performed without nucleic acids and then loaded), line 3 is the result of loading only 5 µl of GAPDH primer (forward primer: TGCACCACCAACTGCTTAGC (SEQ ID NO: 1), reverse primer: CGCATGGACTGTGGTCATGAG (SEQ ID NO: 2)), line 4 is a positive control group (PCR performed for the isolated gDNA (1 µg) using a commercial gDNA isolation kit (gDNA extraction using AccuPrep^{®}Genomic DNA Extraction Kit (K-3032) of Bioneer), line 5 is the result of filtering the mixture in which the sample and the nucleic acid isolation buffer according to an embodiment of the present invention were mixed through a membrane filter, as in Example 2-1, and loading the same (referred to as attach and bind (AB) elution), line 6 is the result of eluting the membrane filter in which the mixture was filtered in line 5 with distilled water, as in Example 2-2, and then loading the distilled water (described as AB (attach and bind)) (the lines 5 and 6 are experimental groups to identify whether the membrane filter binds to gDNA), line 7 is the result of loading the result obtained by applying 2 ml of the buffer composition of Example 1 and performing the same as in Example 2-1 (described as ST (standard concentration)), line 8 is the result of applying 2 ml of high concentration SDS (1%, described as HS (High SDS)), line 9 is the result of applying 2 ml of medium concentration SDS (0.5%, MS (medium SDS)), line 10 is the result of applying 2 ml of high concentration NaCl (1 M, described as HN (High NaCl)), line 11 is the result of applying 2 ml of medium concentration NaCl (0.5 M, described as MN (Medium NaCl)), line 12 is the result of applying 2 ml of high concentration EtOH (70%, described as HE (High EtOH)), and line 13 is the result of applying 2 ml of medium concentration EtOH (50%, described as ME (Medium EtOH)).

As shown in FIG. 2, as a result of identifying whether gDNA was bound to the membrane filter, it was identified that the light emission of line 6 was clear compared to line 5, so the nucleic acids were well bound to the membrane filter *(see* line 5), and that distilled water was used for excellent isolation of nucleic acids therefrom *(see* line 6).

In addition, only when EtOH was 50% or less, PCR applying a membrane filter could be performed immediately without an elution process *(see* line 13). In addition, in the case of SDS, it was identified that PCR was not performed when 1% SDS was included (*see* line 8).

Accordingly, in the nucleic acid isolation method using the nucleic acid isolation buffer composition of an embodiment of the present invention and directly applying the membrane filter on which the nucleic acids are immobilized, excluding the elution process, it was identified that it was preferable that the SDS was included in 1% or less, and that it was preferable that the EtOH was included in 50% or less.

### Example 4. Identification of Nucleic Acid Isolation Efficiency by Membrane Filter

### 4-1. Commercial Membrane Filter

Among commercial membrane filters made of a PVDF material having various pore sizes, filters compatible for the nucleic acid isolation buffer composition according to an embodiment of the present invention were identified.

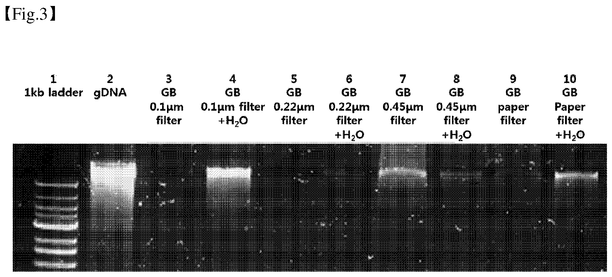

In this regard, a syringe provided with a 0.1 µm PVDF filter, a 0.22 µm PVDF filter, a 0.45 µm PVDF filter or a paper filter (3 M paper filter) and a nucleic acid isolation buffer of an embodiment of the present invention (GB (gDNA extraction buffer) were used. HEK 293T cells (1.0 × 10⁶) were lysed using 5 ml of the nucleic acid isolation buffer according to an embodiment of the present invention. Genomic DNA (gDNA) was isolated in the same manner as in the method of 2-1 of Example 2, and the efficiency of each isolation was identified, as shown in FIG. 3. 5 µl of the sample obtained according to the conditions of each experimental group and 1 µl of the dye were mixed, and loaded at 100 v for 30 minutes using 0.8% agarose gel.

In FIG. 3, line 1 is a 1 kb ladder, line 2 is a gDNA control group (PCR performed for the isolated gDNA (1 µg) using a commercial gDNA isolation kit (gDNA extraction using AccuPrep^{®}Genomic DNA Extraction Kit (K-3032) of Bioneer), line 3 (a filter having a pore size of 0.1 µm), line 5 (a filter having a pore size of 0.22 µm), line 7 (pore size 0.45 µm) and line 9 (paper filter) are experimental groups in which 100 µl of gDNA (10 µg) isolated according to Example 2 and 300 µl of the nucleic acid isolation buffer according to an embodiment of the present invention were filtered through each membrane filter (experimental groups to identify the amount of gDNA remaining in the nucleic acid isolation buffer that is not bound to the membrane after filtration), and line 4 (a filter having a pore size of 0.1 µm), line 6 (a filter having a pore size of 0.22 µm), line 8 (pore size 0.45 µm), and line 10 (paper filter) are experimental groups in which nucleic acids were obtained by eluting with 400 µl of H₂O in the final stage in the method of 2-2 of Example 2. In this connection, in order to filter out impurities in lines 4, 6, 8 and 10, the membrane filter was washed once with 400 µl of 100% EtOH.

As identified in FIG. 3, a clear band was identified in line 4. Accordingly, it was identified that, in commercial membrane filters made of a PVDF material having various pore sizes, the filter having a pore size of 0.1 µm showed the highest DNA isolation efficiency. On the other hand, in the case of the filter having a pore size of 0.45 µm, gDNA was not bound to the membrane filter, so a significant amount of gDNA could be identified in the filtered solution. Accordingly, in the filter made of a PVDF material, it was identified that the filter having a pore size of 0.1 µm was most compatible with the nucleic acid isolation buffer composition of an embodiment of the present invention.

### 4-2. Silica Membrane Filter

The compatibility between the silica membrane filter and the nucleic acid isolation buffer composition according to an embodiment of the present invention was also evaluated.

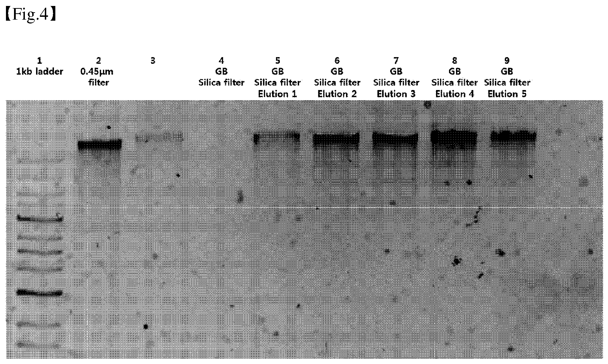

In this regard, Genomic DNA (gDNA) was isolated from 293T cells in the same manner as in the method of 2-1 of Example 2 using an injection provided with a silica filter, and the efficiency of each isolation was identified, as shown in FIG. 4. In this connection, the amount of eluted nucleic acid was identified according to the amount of the elution solution (described as silica filter elution 1 to 5).

In FIG. 4, line 1 is a 1 kb ladder, line 2 is a positive control group (an experimental group in which after mixing gDNA (1 µg) isolated using a commercial gDNA isolation kit (gDNA extraction using AccuPrep^{®}Genomic DNA Extraction Kit (K-3032) of Bioneer) and 400 µl of the nucleic acid isolation buffer of an embodiment of the present invention, the mixture was filtered through a 0.1 µM PVDF membrane filter, and then the membrane filter was eluted with 200 µl of distilled water), line 3 is an experimental group in which the membrane filter of line 2 eluted once was eluted again using 200 µl of distilled water (an experimental group to identify that there is no remaining gDNA after elution), line 4 is an experimental group using the nucleic acid isolation buffer of an embodiment of the present invention containing 1 µg of gDNA after filtering the same through a silica membrane filter (an experimental group to identify whether gDNA is bound to the silica membrane filter), line 5 is an experimental group in which of the silica membrane filter was eluted with 50 µl of distilled water, line 6 is an experimental group in which the silica membrane filter was eluted twice repeatedly with 50 µl of distilled water, line 7 is an experimental group in which the silica membrane filter was eluted 3 times repeatedly with 50 µl of distilled water, line 8 is an experimental group in which the silica membrane filter was eluted 4 times repeatedly with 50 µl of distilled water, and line 9 is an experimental group in which the silica membrane filter was eluted 5 times repeatedly with 50 µl of distilled water.

Accordingly, as shown in FIG. 4, it was identified that the gDNA isolation efficiency was excellent even when the nucleic acid isolation buffer composition of an embodiment of the present invention and the silica membrane filter were used. In particular, a clear band was identified in lines 5 to 9, and as a reference, it was identified that the volume of the elution solution of about 100 to 200 µl was appropriate.

### 4-3. Glass Microfiber Filter

### 1) Identification of Compatibility

The compatibility between the glass fiber filter and the nucleic acid isolation buffer composition according to an embodiment of the present invention was also evaluated.

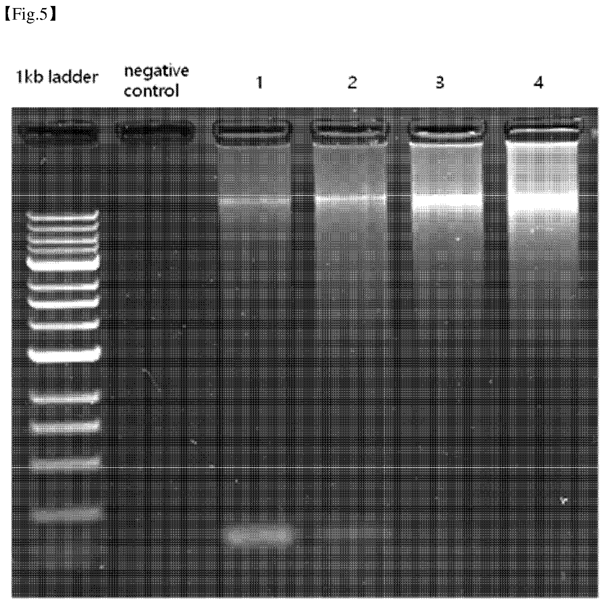

In this regard, Genomic DNA (gDNA) was isolated from 293T cells in the same manner as in the method of 2-1 of Example 2 using a syringe provided with a glass fiber filter, and the efficiency of each isolation was identified, as shown in FIG. 5.

In FIG. 5, line 1 is a 1 kb ladder, line 2 is a negative control, and is an experimental group in which 200 µl of the nucleic acid isolation buffer composition according to an embodiment of the present invention was added without gDNA, lines 3 and 4 are experimental groups in which the nucleic acid isolation buffer of an embodiment of the present invention containing 1µg of gDNA was filtered with a glass fiber filter and then eluted with 50 µl of distilled water under the same conditions twice repeatedly, line 5 is an experimental group in which the glass fiber filter was eluted with 100 µl of distilled water, and line 6 is an experimental group in which the glass fiber filter was eluted with 200 µl of distilled water.

As shown in FIG. 5, since lines 1 to 4, which are the experimental groups using the glass fiber filter, showed a clear band, it was identified that gDNA was isolated. Accordingly, it was identified that the gDNA isolation efficiency was excellent even when the nucleic acid isolation buffer composition of an embodiment of the present invention and the glass fiber filter were used.

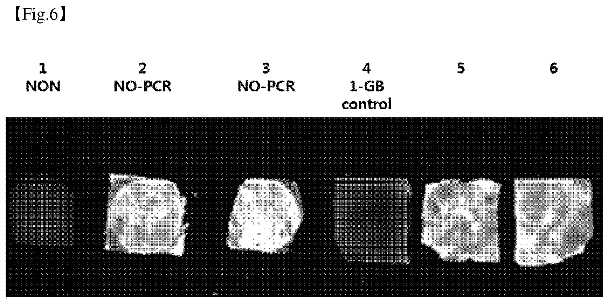

In addition, it is shown in FIG. 6 by visually identifying whether the glass fiber filter is immobilized on gDNA. To this end, after treating the glass fiber filter with EtBr (Ethidium bromide), the light emission under UV was identified to determine whether the glass fiber filter was immobilized on gDNA, which is shown in FIG. 6.

In FIG. 6, lines 1 to 4 are control groups, and are experimental groups in which 200 µl of the nucleic acid isolation buffer composition according to an embodiment of the present invention was added without gDNA, lines 2 and 3 are experimental groups performed repeatedly under the same conditions, and are experimental groups in which PCR was not performed, and lines 5 and 6 are experimental groups performed repeatedly under the same conditions, and are experimental groups in which PCR was performed.

From the result of FIG. 6 identifying the glass fiber membrane filter to which gDNA is bound by UV image without an elution process, it was identified that the gDNA was well bound to the glass fiber membrane filter because a clear band light emission was observed in lines 2 and 3. In addition, in the case of PCR immediately excluding the elution process using a glass fiber membrane filter, as a result of identifying the remaining amount of gDNA in the glass fiber membrane filter, it was identified that it was slightly reduced compared to before when PCR was performed. This was determined to indicate that some gDNA was lysed in the PCR reaction mixture during a PCR process.

Accordingly, it was identified that the gDNA binding and isolation efficiency were excellent even when the nucleic acid isolation buffer composition of an embodiment of the present invention and the glass fiber membrane filter were used.

### 2) Identification of Optimal Size

The negatively charged membrane filters, such as PVDF membrane, silica membrane and glass fiber membrane filters, require a large amount of salt(0.1 to 1 M NaCl) as an intermediate medium of positive charge to capture negatively charged gDAN. Accordingly, since the glass fiber filter including nucleic acids contains a large amount of Na+, which is a gDNA binding mediator, when PCR is performed without separate purification, it may cause inhibition of the PCR reaction. The most efficient method to prevent the same is to minimize the effect of Na+ by making the glass fiber filter as small as possible compared to the PCR volume. However, there is an attention point that when it is made too small, it is difficult to manufacture a column-based nucleic acid isolation kit provided with a glass fiber membrane filter. Accordingly, the optimal size of the glass fiber filter was identified in the utilization with the nucleic acid isolation buffer composition of an embodiment of the present invention.

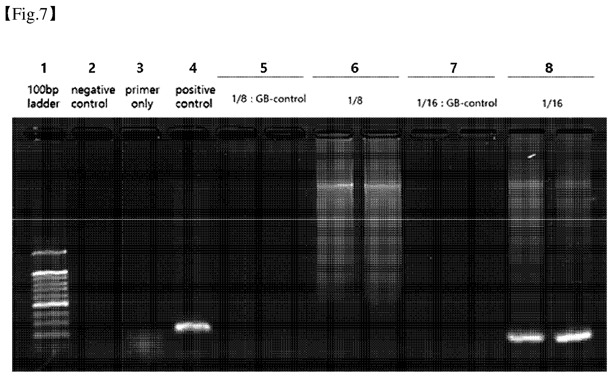

Genomic DNA (gDNA) was isolated from 293T cells in the same manner as in the method of 2-1 of Example 2, and the efficiency of each isolation was identified, as shown in FIG. 7.

In this connection, 0.8% of agarose gel was used to identify the PCR product, and it was loaded at 100 v for 30 minutes. In addition, PCR was performed in a total volume of 50 µl, and 5 µl of the sample and 1 µl of the dye were mixed and loaded.

In FIG. 7, line 1 is a 100 bp ladder (2 µl of ladder + 3 µl of H₂O), line 2 is a negative control group, and is an experimental group in which only 200 µl of the nucleic acid isolation buffer composition according to an embodiment of the present invention was added without gDNA, line 3 is an experimental group in which only 5 µl of a primer for GAPDH (forward primer: TGCACCACCAACTGCTTAGC (SEQ ID NO: 1), reverse primer: CGCATGGACTGTGGTCATGAG (SEQ ID NO: 2)) was loaded, line 4 is a positive control group (PCR performed for the isolated gDNA (1 µg) using a commercial gDNA isolation kit (gDNA extraction using AccuPrep^{®}Genomic DNA Extraction Kit (K-3032) of Bioneer), line 5 is an experimental group in which PCR was performed with the filtered buffer after filtering the nucleic acid isolation buffer of an embodiment of the present invention containing 1 µg of gDNA with the glass fiber filter (a control group of line 6), line 6 is an experimental group in which 400 µl of the nucleic acid isolation buffer of an embodiment of the present invention containing 1 µg of gDNA was filtered through the glass fiber filter, cut into 1/8 size (4 × 4 mm²), and then subjected to PCR reaction, line 7 is an experimental group in which 400 µl of the nucleic acid isolation buffer of an embodiment of the present invention containing 1 µg of gDNA was filtered through the glass fiber filter, and the filtered buffer was subjected to PCR (a control group of line 8), and line 8 is an experimental group in which 400 µl of the nucleic acid isolation buffer of an embodiment of the present invention containing 1 µg of gDNA was filtered through the glass fiber filter, cut to 1/16 size (2 × 2 mm²), and then subjected to PCR reaction.

As shown in FIG. 7, based on a total volume of 50 µl of PCR reaction, a clear band was observed in line 8, identifying that PCR was successfully performed at a size of 2 × 2 mm² (1/16). On the other hand, as shown in line 6, it was identified that PCR was not performed at a size of 4 × 4 mm² (1/8). Accordingly, it was identified that the glass fiber filter per 50 µl of total volume was optimal at a size of 2 × 2 mm² or less (based on 0.25 M NaCl).

### Example 5. Identification of Nucleic Acid Isolation Efficiency of One-Step Nucleic Acid Isolation Method

### 5-1. Identification of Sensitivity according to Amount of gDNA

In the nucleic acid isolation method using the nucleic acid isolation buffer composition of an embodiment of the present invention and directly applying the membrane filter on which nucleic acids are immobilized to PCR, excluding an elution process, it was identified whether a small amount of gDNA could be detected by PCR. When a glass fiber filter is used, the sensitivity according to the amount of gDNA (µg (microgram) to pg (picogram) unit) needs to be verified.

To this end, DNA was extracted from 293T cells using a commercial gDNA isolation kit (gDNA extraction using AccuPrep^{®}Genomic DNA Extraction Kit (K-3032) of Bioneer). A total of 120 µg of the extracted gDNA was mixed with 400 µl of the nucleic acid isolation buffer composition according to an embodiment of the present invention, and then 40 µl, which is 1/10 thereof, was mixed with 360 µl of the nucleic acid isolation buffer composition according to an embodiment of the present invention. Serial dilution was performed 7 times, in which 1/10 of the mixed solution was diluted again with the nucleic acid isolation buffer composition according to an embodiment of the present invention, and then each was filtered through the glass fiber filter. Thereafter, the filter was cut into 2 × 2 mm² and put into a PCR reaction mixture to identify whether PCR amplification was performed.

In this connection, 0.1% of agarose gel was used and loaded at 100 v for 30 minutes. In addition, PCR was performed in a total volume of 50 µl, and 5 µl of the sample and 1 µl of the dye were mixed and loaded.

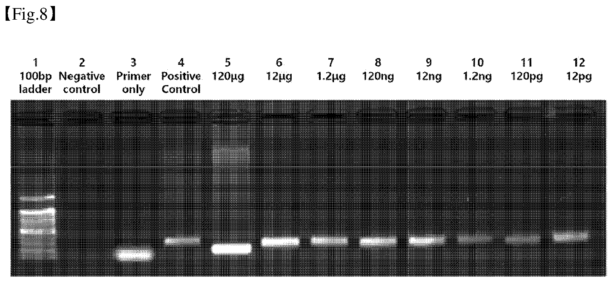

In FIG. 8, line 1 is a 100 bp ladder, line 2 is a negative control group, and an experimental group in which only 200 µl of the nucleic acid isolation buffer composition according to an embodiment of the present invention was added without gDNA, line 3 is an experimental group in which only 5 µl of a primer for GAPDH (forward primer: TGCACCACCAACTGCTTAGC (SEQ ID NO: 1), reverse primer: CGCATGGACTGTGGTCATGAG (SEQ ID NO: 2)) was loaded, line 4 is a positive control group (PCR performed for the isolated gDNA (1 µg) using a commercial gDNA isolation kit (gDNA extraction using AccuPrep^{®}Genomic DNA Extraction Kit (K-3032) of Bioneer), line 5 is an experimental group in which 120 µg of gDNA was immobilized on the glass fiber filter, cut the same into a size of 2 × 2 mm² and put the same in the PCR reaction mixture to identify whether PCR amplification was performed, line 6 is the PCR result of 12 µg of gDNA, line 7 is the PCR result of 1.2 µg of gDNA, line 8 is the PCR result of 120 ng of gDNA, line 9 is the PCR result of 12 ng of gDNA, line 10 is the PCR result of 1.2 ng of gDNA, line 11 is the PCR result of 120 pg of gDNA, and line 12 is the PCR result of 12 pg of DNA.

As shown in FIG. 8, a clear band was identified in line 12 (12 pg of gDNA), identifying that PCR amplification was excellent even with a small amount of gDNA.

### 5-2. Identification of Sensitivity according to Number of Cells

In the nucleic acid isolation method using the nucleic acid isolation buffer composition of an embodiment of the present invention and directly applying the membrane filter on which nucleic acids are immobilized to PCR, excluding an elution process, the sensitivity according to the amount of gDNA obtained from the small number of cells needs to be verified. To this end, when a glass fiber filter was used, the sensitivity according to the amount of gDNA of 100 units from 100,000 cells was identified.

To this end, 100,000 of 293T cells were lysed in 400 µl of water, 200 µl thereof was collected and transferred to a new tube, and then 200 µl of water was added and mixed for serial dilution by 1/2 (after dilution, the volume of each tube was 200 µl). Cells were lysed using the nucleic acid isolation buffer composition (GB, gDNA extraction buffer) according to an embodiment of the present invention, eluted through the glass fiber filter. Then, the glass fiber filter cut into a size of 2 × 2 mm² was put into a PCR reaction mixture to identify whether PCR amplification was performed.

In this connection, 0.1% agarose gel was used and loaded at 100 v for 30 minutes. In addition, PCR was performed in a total volume of 50 µl, and 5 µl of the sample and 1 µl of the dye were mixed and loaded.

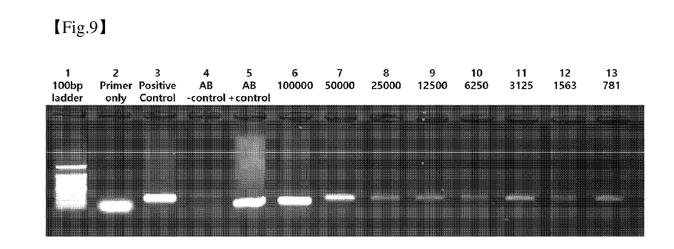

In FIG. 9, line 1 is a 100 bp ladder (2 µl of ladder + 3 µl of H₂O), line 2 is an experimental group in which only a primer for GAPDH (forward primer: TGCACCACCAACTGCTTAGC (SEQ ID NO: 1), reverse primer: CGCATGGACTGTGGTCATGAG (SEQ ID NO: 2)) was loaded, line 3 is a positive control group (PCR performed for the isolated gDNA (1 µg) using a commercial gDNA isolation kit (gDNA extraction using AccuPrep^{®}Genomic DNA Extraction Kit (K-3032) of Bioneer), line 4 is a negative control group (an experimental group containing only 200 µl of the nucleic acid isolation buffer composition according to an embodiment of the present invention without gDNA, AB-control), line 5 is a positive control group (1 µg of qDNA and 200 µl of the nucleic acid isolation buffer composition according to an embodiment of the present invention, AB+control), line 6 is an experimental group of 100,000 cells (200 µl of the nucleic acid isolation buffer composition according to an embodiment of the present invention), line 7 is an experimental group of 50,000 cells (200 µl of the nucleic acid isolation buffer composition according to an embodiment of the present invention), line 8 is an experimental group of 25,000 cells (200 µl of the nucleic acid isolation buffer composition according to an embodiment of the present invention), line 9 is an experimental group of 12,500 cells (200 µl of the nucleic acid isolation buffer composition according to an embodiment of the present invention), line 10 is an experimental group of 6,250 cells (200 µl of the nucleic acid isolation buffer composition according to an embodiment of the present invention), line 11 is an experimental group of 3,125 cells (200 µl of the nucleic acid isolation buffer composition according to an embodiment of the present invention), line 12 is an experimental group of 1,563 cells (200 µl of the nucleic acid isolation buffer composition according to an embodiment of the present invention), and line 13 is an experimental group of 781 cells (200 µl of the nucleic acid isolation buffer composition according to an embodiment of the present invention).

As shown in FIG. 9, it was identified that PCR amplification was successfully performed on gDNA despite the small number of cells of 781 (*see* line 13).

### 5-3. Identification of Bacterial gDNA Isolation Efficiency

In the nucleic acid isolation method using the nucleic acid isolation buffer composition of an embodiment of the present invention and directly applying the membrane filter on which nucleic acids are immobilized to PCR, excluding an elution process, in the case of using a glass fiber filter, it was identified whether the bacterial gDNA having a cell wall could also be isolated.

To this end, 10⁹ of *E. coli* cells and 400 µl of the nucleic acid isolation buffer according to an embodiment of the present invention were mixed and lysed, and then 200 µl thereof was collected and transferred to a new tube, and 200 µl of the nucleic acid isolation buffer according to an embodiment of the present invention was added and mixed for serial dilution by 1/2 (after dilution, the volume of each tube was 200 µl). After elution through the glass fiber filter, the glass fiber filter cut into a size of 2 × 2 mm² was put into a PCR reaction mixture to identify whether PCR amplification was performed.

The primers used for amplification from bacterial gDNA were primers for uidA gene, and a forward primer (uidA Up): TATGGAATTTCGCCGATTTT (SEQ ID NO: 3) and a reverse primer (uidA Down): TGTTTGCCTCCCTGCTGCGG (SEQ ID NO: 4) were used. In addition, the PCR reaction mixture and PCR conditions used are as described in the above Examples.

In this connection, 0.1% agarose gel was used and loaded at 100 v for 30 minutes. In addition, PCR was performed in a total volume of 50 µl, and 5 µl of the sample and 1 µl of the dye were mixed and loaded.

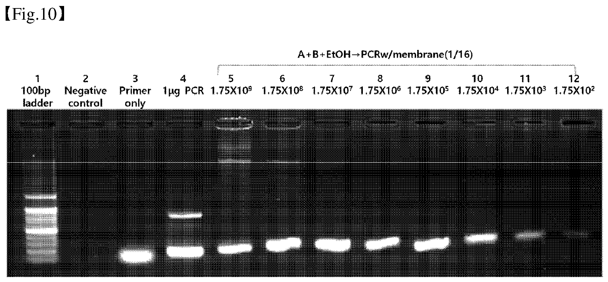

In FIG. 10, line 1 is a 100 bp ladder, line 2 is a negative control group (200 µl of the nucleic acid isolation buffer composition according to an embodiment of the present invention), line 3 is an experimental group in which only 5 µl of primers for the *E. coli* gene were loaded, line 4 is a positive control group (PCR performed for the isolated gDNA (1µg) using a commercial gDNA isolation kit (gDNA extraction using AccuPrep^{®}Genomic DNA Extraction Kit (K-3032) of Bioneer), line 5 is an experimental group of 1.75 × 10⁹ cells (200 µl of the nucleic acid isolation buffer composition according to an embodiment of the present invention), line 6 is an experimental group of 1.75 × 10⁸ *E. coli* cells (200 µl of the nucleic acid isolation buffer composition according to an embodiment of the present invention), line 7 is an experimental group of 1.75 × 10⁷ *E. coli* cells (200 µl of the nucleic acid isolation buffer composition according to an embodiment of the present invention), line 8 is an experimental group of 1.75 × 10⁶ *E. coli* cells (200 µl of the nucleic acid isolation buffer composition according to an embodiment of the present invention), line 9 is an experimental group of 1.75 × 10⁵ *E. coli* cells (200 µl of the nucleic acid isolation buffer composition according to an embodiment of the present invention), line 10 is an experimental group of 1.75 × 10⁴ *E. coli* cells (200 µl of the nucleic acid isolation buffer composition according to an embodiment of the present invention), line 11 is an experimental group of 1.75 × 10³ *E. coli* cells (200 µl of the nucleic acid isolation buffer composition according to an embodiment of the present invention), and line 12 is an experimental group of 1.75 × 10² *E. coli* cells (200 µl of the nucleic acid isolation buffer composition according to an embodiment of the present invention).

As shown in FIG. 10, it was identified that PCR amplification was successfully performed even with the small number of bacterial gDNA of about 175 (*see* line 12).

### 5-4. Identification of RNA Isolation Efficiency according to pH

In the nucleic acid isolation method using the nucleic acid isolation buffer composition of an embodiment of the present invention and using a membrane filter on which nucleic acids are immobilized without an elution process, the total RAN isolation efficiency according to the pH of cells was identified.

In order to isolate total RNA from cells, a change in pH different from DNA is required. Under various pH changes, using the nucleic acid isolation buffer composition according to an embodiment of the present invention and a glass fiber filter, total RNA was isolated and immobilized on the filter, and then eluted to identify total RNA isolation. In this connection, the cells were used as 293T cells, 1 × 10⁶ in the same manner.

The cells were mixed with 2 ml of the nucleic acid isolation buffer composition (GB, gDNA extraction buffer) according to an embodiment of the present invention and incubated for 5 minutes for lysis. Thereafter, total RNA was eluted through a glass fiber filter, immobilized on the glass fiber filter, and eluted with distilled water to identify whether total RNA was extracted. In order to analyze the total RNA extraction efficiency according to pH, 25 mM Tris-HCl of pH4 to pH8 was used.

In this connection, 1% agarose gel was used and loaded at 100 v for 30 minutes. In addition, PCR was performed in a total volume of 50 µl, and 5 µl of the sample and 1 µl of the dye were mixed and loaded.

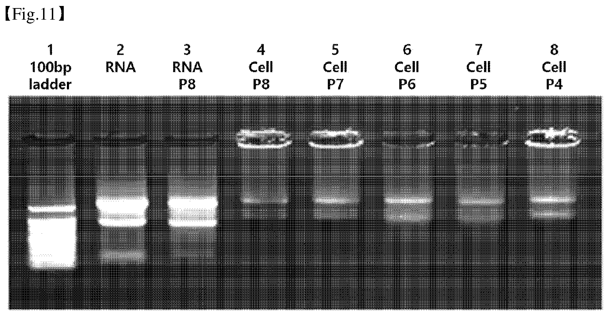

In FIG. 11, line 1 is a 100 bp ladder (2 µl of ladder + 3 µl of H₂O), line 2 is a positive control group (total RNA (1 µg) isolated using a commercial total RNA isolation kit (total RNA extraction using AccuPrep^{®}Universal RNA Extraction Kit of Bioneer)), line 3 is another control group, and an experimental group in which total RNA (1 µg) obtained using a commercial total RNA isolation kit as in line 2 was lysed with the nucleic acid isolation buffer composition of an embodiment of the present invention containing 2 ml of 25 mM Tris-HCl at a pH of 8, and lines 4 to 8 are experimental groups in which cells were lysed with the nucleic acid isolation buffer composition of an embodiment of the present invention containing 25mM Tris-HCl at pH of 4 to 8, respectively.

As shown in FIG. 11, it was identified that total RNA was not isolated well at a pH of 8, which is a pH suitable for gDNA, but total RNA was isolated well at a pH of 7 or less. Accordingly, it was identified that in the total RNA isolation method using the nucleic acid isolation buffer composition and glass fiber filter according to an embodiment of the present invention and using a membrane filter on which nucleic acids were immobilized without an elution process, it was preferable to perform the above at pH of 4 to 7.

### 5-5. Identification of PCR Efficiency after RNA Isolation according to pH

Using the nucleic acid isolation buffer composition according to an embodiment of the present invention and a glass fiber filter, total RNA was isolated from cells and immobilized on a membrane filter, and then the membrane on which the total RNA was immobilized was put into a PCR tube to identify whether a PCR reaction was possible immediately.

Unlike DNA, in order to isolate RNA, it is necessary to change the buffer composition of a pH different from that of DNA. In order to analyze the effect on the PCR reaction after extraction of total RNA according to pH, 25 mM Tris-HCl of pH4 to pH8 was used. Under various pH changes, 1 × 10⁶ of 293T cells were incubated for 5 minutes using 2 ml of the nucleic acid isolation buffer composition according to an embodiment of the present invention for lysis. Total RNA was eluted through a glass fiber filter, immobilized on the glass fiber filter, cut into 2 × 2 mm², and put into a PCR reaction mixture to identify whether PCR amplification was performed.

In this connection, 1% agarose gel was used and loaded at 100 v for 30 minutes. In addition, PCR was performed in a total volume of 50 µl, and 5 µl of the sample and 1 µl of the dye were mixed and loaded.

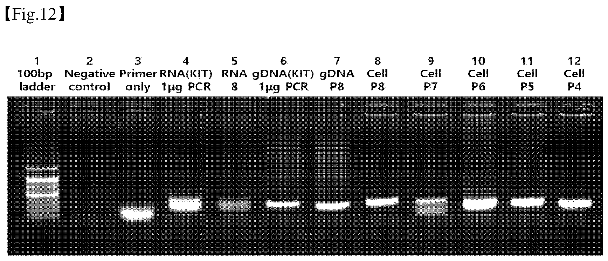

In FIG. 12, line 1 is a 100 bp ladder (2 µl of ladder + 3 µl of H₂O), line 2 is a negative control group, and an experimental group containing only 200 µl of the nucleic acid isolation buffer composition according to an embodiment of the present invention, line 3 is the result of loading only 5 µl of GAPDH primer (forward primer: TGCACCACCAACTGCTTAGC (SEQ ID NO: 1), reverse primer: CGCATGGACTGTGGTCATGAG (SEQ ID NO: 2)), line 4 is a positive control group (total RNA (1 µg) isolated using a commercial total RNA isolation kit (total RNA extraction using AccuPrep^{®}Universal RNA Extraction Kit of Bioneer)), line 5 is an experimental group in which total RNA (1 µg) obtained using a commercial total RNA isolation kit as in line 4 was lysed with the nucleic acid isolation buffer composition of an embodiment of the present invention containing 2 ml of 25 mM Tris-HCl at a pH of 8, line 6 is a positive control group (PCR performed for the isolated gDNA (1 µg) using a commercial gDNA isolation kit (gDNA extraction using AccuPrep^{®}Genomic DNA Extraction Kit (K-3032) of Bioneer), line 7 is an experimental group in which gDNA (1 µg) obtained using a commercial gDNA isolation kit as in line 6 was lysed with the nucleic acid isolation buffer composition of an embodiment of the present invention containing 2 ml of 25 mM Tris-HCl at a pH of 8, and lines 8 to 12 are experimental groups in which cells were lysed with the nucleic acid isolation buffer composition of an embodiment of the present invention containing 25mM Tris-HCl at pH of 4 to 8, respectively.

As shown in FIG. 12, it was identified that after lysing the cells with the nucleic acid isolation buffer composition of an embodiment of the present invention containing 25 mM Tris-HCl at pH of 4 to 7, even when it was filtered through a glass fiber filter and PCR was performed directly into a PCR tube, it was performed successfully.

Comprehensively, with the nucleic acid isolation buffer composition and the nucleic acid isolation kit in which a membrane filter including the same is provided according to an embodiment of the present invention, a PCR reaction can be performed in a one-tube, and the isolation of nucleic acids from various samples can be performed in one-step. In addition, it was identified that the nucleic acid isolation buffer composition had excellent compatibility with membrane filters of various materials. In addition, in the nucleic acid isolation method to which the same is applied, it was identified that the method enables the direct application of the membrane filter, on which nucleic acids are immobilized, to PCR without a separate elution process. Accordingly, the nucleic acid isolation composition, the kit and the isolation method according to an embodiment of the present invention may be effectively used in rapid diagnosis onsite.

## Claims

1. A one-step nucleic acid isolation buffer composition comprising ethanol, sodium dodecyl sulfate (SDS), sodium chloride (NaCl), ethylenediaminetetraacetic acid (EDTA), and Tris-HCl.

2. The composition of claim 1, wherein the buffer composition comprises 20% (v/v) to 50% (v/v) of ethanol, 0.1% (w/v) to 0.5% (w/v) of SDS, 0.5 M to 1 M of sodium chloride (NaCl), 1 mM to 100 mM of EDTA, and 1 mM to 50 mM of Tris-HCl.

3. A one-step nucleic acid isolation kit comprising the nucleic acid isolation buffer composition according to any one of claims 1 and 2 and a column in which a membrane filter is provided.

4. The kit of claim 3, wherein the membrane filter is any one selected from the group consisting of polyvinylidene fluoride (PVDF), nylon filter, cellulose nitrate filter, paper filter, glass fiber filter, and silica filter.

5. The kit of claim 3, wherein the kit is in a form of a syringe.

6. The kit of claim 3, wherein the nucleic acid is at least one selected from the group consisting of DNA, RNA and PNA.

7. A method for isolating nucleic acids from a sample using the one-step nucleic acid isolation kit according to claim 3.

8. The method of claim 7, wherein the method comprise:
dissolving a sample by immersing the same in the nucleic acid isolation buffer according to any one of claims 1 and 2;
incubating a solution in which the sample is dissolved at room temperature;
transferring the solution in which the sample is dissolved to a column provided with a membrane filter; and
applying pressure to the column to immobilize nucleic acids on a filter and removing a filtrate.

9. The method of claim 7, wherein the method comprise:
dissolving a sample by immersing the same in the nucleic acid isolation buffer according to any one of claims 1 and 2;
incubating a solution in which the sample is dissolved at room temperature;
transferring the solution in which the sample is dissolved to a column provided with a membrane filter;
applying pressure to the column to immobilize nucleic acids on a filter and removing a filtrate;
putting an elution solution in the column on which the nucleic acids are immobilized and passing the same through the membrane filter; and
obtaining the elution solution.

10. The method of claim 9, wherein the elution solution is any one selected from the group consisting of distilled water, TE buffer (T ten E one buffer), and a mixture thereof.

11. The method of claim 8, wherein the incubation is performed for 2 to 10 minutes.

12. The method of claim 7, wherein the sample is a biological or non-biological sample.

13. The method of claim 12, wherein the biological sample is at least one selected from the group consisting of nasal aspirate, bronchial aspirate, organ secretions, sputum, tears, saliva, cell, cell extract, whole blood, plasma, serum, mucus, nasal washes, urine, semen, peritoneal washings, ascites, cystic fluid, meningeal fluid, amniotic fluid, leukocytes, peripheral blood mononuclear cells, buffy coat, glandular fluid, pancreatic fluid, lymph fluid, pleural fluid, nipple aspirate, synovial fluid, joint aspirate, and cerebrospinal fluid.

14. The method of claim 12, wherein the non-biological sample comprises chemically synthesized PNA.

15. A method for amplifying nucleic acids, the method comprising performing PCR using the nucleic acids isolated by the method according to claim 7 as a template.

16. The method of claim 15, wherein the PCR is any one selected from the group consisting of a polymerase chain reaction (PCR), a reverse transcription polymerase chain reaction (RT-PCR), and an isothermal amplification PCR.
